Europäisches Patentamt
European Patent Office
Office européen des brevets

(19)

(11) Publication number: 0 261 679 A2

(12) # EUROPEAN PATENT APPLICATION

(21) Application number: 87113969.7

(22) Date of filing: 24.09.87

(51) Int. Cl.⁴: C08G 8/36 , C07C 43/23 , C07C 41/03 , C09D 3/48 , C09D 5/44

(30) Priority: 24.09.86 JP 226897/86
24.09.86 JP 226898/86
24.09.86 JP 226899/86

(43) Date of publication of application: 30.03.88 Bulletin 88/13

(84) Designated Contracting States: DE FR GB IT SE

(71) Applicant: Nippon Paint Co., Ltd.
2-1-2, Oyodokita Oyodo-ku
Osaka-shi Osaka-fu(JP)

(72) Inventor: Tsuchiya, Yasuyuki
3-8-23, Kuzuhaasahi
Hirakata-shi Osaka-fu(JP)
Inventor: Tobinaga, Kenshiro
2-32-5, Yuyamadai
Kawanishi-shi Hyogo-ken(JP)
Inventor: Obika, Toshimi
5-9-10, Sagisu Fukushima-ku
Osaka-shi Osaka-fu(JP)
Inventor: Sakamoto, Hiroyuki
7-9, Kitashowa-cho
Nishinomiya-shi Hyogo-ken(JP)
Inventor: Morita, Kengi
4-25-1, Kishibekita
Suita-shi Osaka-fu(JP)
Inventor: Tsushima, Hiroshi
2-17, Kinmitsu-cho
Ashiya-shi Hyogo-ken(JP)

(74) Representative: Hansen, Bernd, Dr.rer.nat. et al
Hoffmann, Eitle & Partner Patentanwälte
Arabellastrasse 4
D-8000 München 81(DE)

(54) **Phenol ether compound production and usage thereof.**

(57) Disclosed is a phenol ether characterized by a beta-hydroxyl group and has the following formula:

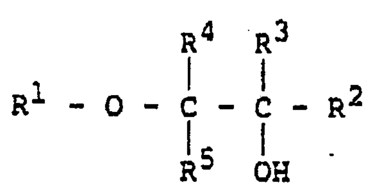

(1)

wherein $R^1$ is a residue of an initial reaction product of a resol type phenol resin from which a phenolic



Xerox Copy Centre

hydroxyl group is removed, $R^2$, $R^3$, $R^4$ and $R^5$ are respectively a saturated or unsaturated alkyl group having 1 to 30 carbon atoms, a saturated or unsaturated cycloalkyl group having 1 to 30 carbon atoms, or a substituted or unsubstituted aryl group having 6 to 20 carbon atoms ($R^3$ and $R^4$ may constitute a ring together).

Also, the present invention is to provide a process for preparing the phenol ether mentioned above.

Further, the present invention is to provide a cationic or anionic electrocoating composition containing the phenol ether containing a beta-hydroxyl group.

# PHENOL ETHER COMPOUND, PRODUCTION AND USAGE THEREOF

## Field of the Invention

The present invention relates to a self-curable and reactive phenol ether having a methylol group and a hydroxyl group at the beta-position with respect to an ether bond, a production and usage thereof.

## Background of the Invention

Phenol resins have been widely used for many applications. They, however, are short of water-resistance if a phenolic hydroxyl group remains therein. In order to obviate the problem, the phenolic hydroxyl group is etherified so as to enhance water-resistance. For example, "Phenol Resin" by Shinichi Murade, Nikkan Kogyo Shinbun Company, 1970, discloses an etherification process wherein phenol is reacted with formalin at a low temperature in the presence of an alkaline catalyst to form a sodium salt of dimethylol phenol or trimethylol phenol, which is then desalted by adding a chloride such as an alkyl halide, an aralkyl halide, epihalohydrin and the like. The reason why phenol is preliminary reacted with formalin to form a methylol group is that, if the hydroxyl group of phenol is preliminary etherified, the reactivity of the ortho-or para-position of a benzene ring is not activated because of the absence of the hydroxyl group to become difficult in reaction with formaldehyde. However, the methylol phenol ether obtained by the process mentioned above has stable methylol groups which are hard to cure.

## Summary of the Invention

The present invention is to provide a phenol ether having enhanced reactivity. The phenol ether is characterized by a beta-hydroxyl group and has the following formula:

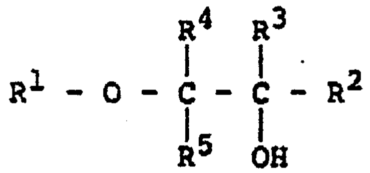

(1)

wherein $R^1$ is a residue of an initial reaction product of a resol type phenol resin from which a phenolic hydroxyl group is removed, $R^2$, $R^3$, $R^4$ and $R^5$ are respectively a saturated or unsaturated alkyl group having 1 to 30 carbon atoms, a saturated or unsaturated cycloalkyl group having 1 to 30 carbon atoms, or a substituted or unsubstituted aryl group having 6 to 20 carbon atoms ($R^3$ and $R^4$ may constitute a ring together).

Also, the present invention is to provide a process for preparing the phenol ether mentioned above.

Further, the present invention is to provide a cationic or anionic electrocoating composition containing the phenol ether containing a beta-hydroxyl group.

## Detailed description of the Invention

The phenol ether of the present invention can be prepared by reacting an initial reaction product of a resol type phenol resin with a monoepoxy compound in the presence of a basic catalyst. The obtained compound (1) has a hydroxyl group at a beta-position with respect to an ether bond and a methylol group, and is self-curable.

By the initial reaction product of a resol type phenol resin is meant a mixture of methylol phenols prepared by reacting a phenol and formaldehyde in the presence of an alkaline catalyst. Examples of the phenols are 3,5-xylenol, m-cresol, 2,3,5-trimethylphenol, 3,4-xylenol, 2,5-xylenol, p-cresol, sarigenin, o-cresol, 2,6-xylenol, p-t-butylphenol and the like. The initial reaction product of the resol type phenol resin preferably has a number average molecular weight of less than 300, which imparts good curing properties. Suitable examples of the initial reaction product of the resol type phenol resin are Tamanol 721 and Tamanol 722, commercially available from Arakawa Chemical Company.

The monoepoxy compound are those having one epoxy group in a molecule, which is generally represented by the following formula:

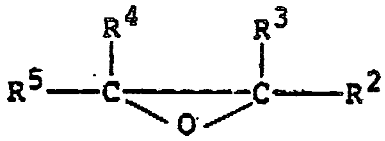

wherein $R^2$, $R^3$, $R^4$ and $R^5$ are respectively a saturated or unsaturated alkyl group having 1 to 30, preferably 1 to 20 carbon atoms, a saturated or unsaturated cycloalkyl group having 1 to 30, preferably 1 to 20 carbon atoms, or a substituted or unsubstituted aryl group having 6 to 20 carbon atoms ($R^3$ and $R^4$ may constitute a ring together). A compound having at least two expoxy groups, such as a diepoxy compound or a triepoxy compound, can be used as a portion of the monoepoxy compound in an amount sufficient to avoid gellation. If necessary, the diepoxy compound or triepoxy compound may be modified by an organic acid or a secondary amine to a monoepoxy compound. Preferred monoepoxy compound is a monoglycidyl compound. Examples of the monoglycidyl compounds are glycidyl ethers, such as phenyl glycidyl ether, butyl glycidyl ether, allyl glycidyl ether, 2-ethylhexyl glycidyl ether, p-t-phenyl glycidyl ether, a glycidyl ether of higher aliphatic alcohol, a glycidyl ether of ethylene oxide additive of lauryl alcohol, a glycidyl ether of ethylene oxide additive of phenol, dibromophenyl glycidyl ether, pentyl glycidyl ether and the like; glycidyl esters, such as glycidyl ester of benzoic acid, a glycidyl ester of a mixture of linolic acid and oleic acid, a glycidyl ester of tertiary saturated carboxylic acid and the like; glycidyl imides, such as glycidyl phthalimide; a monoglycidyl compound prepared by modifying polyglycidyl compound, such as a monoacetate of 1,6-hexanediol diglycidyl ether and the like. Other examples of the monoepoxy compounds are styrene oxide, cyclohexene oxide, ethylene oxide, propylene oxide, butadiene monooxide, isobutylene oxide, methylstyrene oxide, vinylcyclohexene monooxide and the like.

An amount ratio of the initial reaction product of the resol type phenol resin and a monoepoxy compound may be from 1.0/0.5 to 1.0/2.0, preferably from 1.0/0.7 to 1.0/1.5, expressed in terms of phenolic hydroxyl group/epoxy group. If the phenolic hydroxyl group remains in a relatively large amount, the water-resistance of the cured material is liable to decline. Accordingly, the monoepoxy compound may be employed in a larger amount than that of the initial reaction product of the phenol resin. Ratios less than 1.0/0.5 deteriorate the water-resistance of the cured material. When the ratio is more than 1.0/2.0, the epoxy compound remains in a relatively large amount to consume a methylol group, so that the curing properties is deteriorated.

The reaction may preferably be conducted in a solvent for facilitating the reaction. Since the initial reaction product of the resol type phenol resin essentially contains unreacted phenol, formaldehyde and water, it is preferred that the solvent for the reaction has a hydrophilic group. Suitable solvents are alcoholic solvents, such as methanol, ethanol, n-propanol, isopropanol, n-butanol, methoxybutanol, isobutanol, sec-butanol, tert-butanol, n-amyl alcohol, isoamyl alcohol, sec-amyl alcohol, 3-pentanol, tert-amyl alcohol, n-hexanol, cyclohexanol, 2-methylcyclohexanol, benzyl alcohol, furfuryl alcohol, tetrahydrofurfuryl alcohol, abietyl alcohol, ethylene glycol, ethylene glycol menomethyl ether, ethylene glycol monoethyl ether, ethylene glycol isopropyl ether, ethylene glycol monobutyl ether, ethylene glycol isoamyl ether, ethylene glycol monophenyl ether, ethylene glycol benzyl ether, ethylene glycol monoacetate, diethylene glycol, diethylene glycol monomethyl ether, diethylene glycol monoethyl ether, diethylene glycol monobutyl ether, diethylene glycol acetate, triethylene glycol, triethylene glycol monomethyl ether, triethylene glycol monoethyl ether, polyethylene glycol, polypropylene glycol, propylene glycol, monoethyl ether, propylene glycol monobutyl ether and the like. Other solvents, such as ketones, esters and the like can be mixed with the alcoholic solvent. The reaction can be conducted at a temperature of 60 to 130 °C, preferably 70 to 125 °C. If the temperature is more than 130 °C, a condensation reaction of methylol groups takes precedence of the etherification reaction. Temperatures of less than 60 °C does not provide a sufficient reaction rate.

The reaction can be conducted in the presence of an alkaline catalyst, such as sodium hydroxide, potassium hydroxide, amines such as trimethylamine, triethylamine, tri-n-butylamine, triamylamine, dimethylbenzylamine, diethylbenzylamine, dimethylaniline, diethylanilin, N,N-di-n-butylaniline, N,N-diamylaniline, N,N-di-t-amylaniline, triethanolamine, dimethylethanolamine, diethylethanolamine, ethyldiethanolamine, n-butyldiethanolamine, di-n-butylethanolamine, triisopropanolamine, alpha-picoline, beta-picoline, gamma-picoline, 2,4-lutidine, 2,6-lutidine, ethylmorpholine, diethylamine, diisopropylamine, diisobutylamine, diamylamine, N-methylethanolamine, N-ethylethanolamine, diethanolamine, pyridine, lauryltrimethylammonium chloride and the like. Preferred are amines.

The obtained reaction product is generally a mixture containing unreacted reagents and the phenol ether of the present invention. The reaction product containing the phenol ether of the present invention can be used neat for various applications. For example, the phenol ether of the present invention can be employed as a cross-linking agent for an aqueous coating composition, especially an cationic or anionic coating composition; or a solvent based coating composition.

For the cationic aqueous coating composition, a cationic aqueous resin is employed. The cationic aqueous resin can be any type of resins known to the art, for example, a sulfonium group-containing resin, a phosphonium group-containing resin and an amino group-containing resin, such as an amine-modified epoxy resin (Japanese Patent Publication (examined) No. 4978/1979 and 47143/1978, Japanese Patent Publication (unexamined) No. 93024/1979, 8673/1978, 80436/1980 and 206442/1984), an amine-modified polyurethane polyol resin (Japanese Patent Publication (unexamine) No. 15449/1979 and 115476/1980), an amine-modified polybutadiene resin (Japanese Patent Publication (unexamine) No. 16048/1978, 142444/1978 and 90273/1985). For the anionic aqueous coating composition, an anionic aqueous resin is employed. The anionic aqueous resin can be any type of resins known to the art, for example, a maleic oil, a vinyl-modified maleic oil, a cyclopentadiene-modified maleic oil, a carboxyl group-containing epoxy resin, a maleic polybutadiene, and alkyd resin, a carboxyl group-containing acryl resin (Japanese Patent Publication (examine) No. 4058/1974, 21691/1972 and 9096/1973, Japanese Patent Publication (unexamine) No. 41425/1974 and 24231/1974) and the like. The cationic or anionic resin at present in the aqueous coating composition in an amount of 3 to 1,000 parts by weight, preferably 10 to 200 parts by weight based on the amount of the phenol ether of the present invention. Amounts less than 3 parts by weight decline paint stability, and amounts more than 1,000 parts by weight deteriorate the technical effect of the phenol ether in curing properties.

The anionic or cationic resin is neutralized with a base or acid to make water-dispersible or water-soluble. Examples of the acids are mineral acids, such as boric acid, hydrochloric acid, phosphoric acid, sulfuric acid and the like; and organic acids, such as lactic acid, formic acid, propionic acid, butylic acid and the like. Examples of the bases are ammonia; amines, such as monoethyleamine, diethylamine, triethylamine, n-butylamine, di-n-butylamine, N-methylethanolamine, N-ethylethanolamine, dimethylethanolamine, diethanolamine, morpholine; solium hydroxide; potassium hydroxide; and the like.

The aqueous coating composition is generally water dispersion. Another solvent, such as hydrocarbons, alcohols, esters, ethers and ketones may be added to the water dispersion, if necessary. An amount of the solvent is not limited, but generally within the range of about 0.1 to 40 % by weight, preferably within the range of about 0.5 to 25 % by weight.

Although the phenol ether of the present invention is self-curable, another curing agent, for example, melamine resin, urea resin, a blocked polyisocyanate compound or an ester-exchanging agent, may be employed to obtain enhanced curing degree. Such a curing agent may be present in the aqueous coating composition in an amount of 1 to 200 parts by weight based on 10 parts by weight of the phenol ether of the present invention.

The aqueous coating composition of the present invention may further contain a curing catalyst, such as an organic acid salt of cobalt, manganese, iron, zinc, lead, calcium or zirconium; manganese dioxide; a mixture thereof. The curing catalyst, when it is calculated in terms of an amount of metal of the curing catalyst, may be present in the aqueous coating composition in an amount of about 0.005 to 2.0 % by weight, preferably 0.05 to 1.0 % by weight based on the solid content.

The aqueous coating composition of the present invention may contain an extender pigment, such as aluminum silicate, precipitated barium sulfate, coaline and precipitated calcium carbonate; a coloring pigment, such as titanium oxide, carbon black, zinc white, iron oxide red, manganese dioxide and the like; a corrosion preventive pigment, such as strontium chromate, lead chromate, basic lead silicate, aluminum molybdate and the like.

The aqueous coating composition of the present invention may contain an additive, such as a surfactant, a flow controlling agent, an ultraviolet absorber and the like.

The aqueous coating composition of the present invention can be applied to a substrate by spray coating, dipping or electrocoating. An electrocoating method can be known to the art. The applied voltage may be varied greatly and can be, for example, as low as one volt to as high as several thousand volts, but typically is between about 50 to 500 volts. The current density is usually between 1.0 ampere and 15 amperes per square foot and tend to decrease during electrodeposition indicating the formation of a self-insulating film.

The substrate to be used for the electrocoating composition can be one having electroconductivity, such as steel, aluminum, copper, magnesium, metallized plastic, electroconductive carbon.

The phenol ether of the present invention is easily hydrolyzed because of the presence of a hydroxyl group at the beta-position of an ether bond, to reproduce a phenolic hydroxyl group which activates the methylol group to facilitate a condensation reaction of methylol groups. The phenol ether can be acted as a cross-linking agent in a coating composition to enhance curing properties.

## Brief Explanation of the Drawings

Fig.1 shows a $^{13}C$ NMR chart of the obtained compound of Example 1.

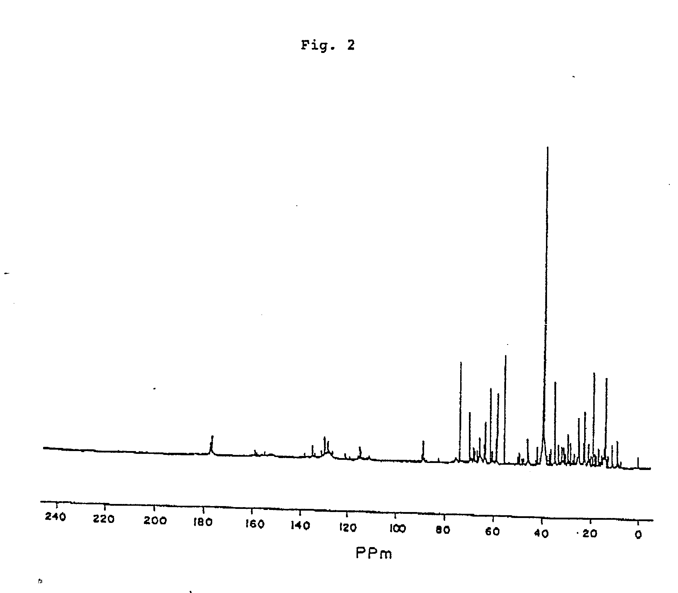

Fig.2 shows a $^{13}C$ NMR chart of the obtained compound of Example 2.

## Examples

The present invention illustrates the following examples, which, however, are not to be construed as limiting the invention to their details.

### Examples 1

A reaction vessel was charged with 60 parts by weight of an initial reaction product of a resol type phenol resin (available from Arakawa Chemical Company as Tamanol 722), to which 10 parts by weight of n-butanol and 10 parts by weight of methoxybutanol were added. After further adding 23 parts by weight of butyl glycidyl ether, the mixture was heated to 100 °C with stirring and then 0.4 parts by weight of dimethylbenzylamine was added. While keeping at a temperature of 100 °C, the mixture was allowed to stand for three hours. An amount of epoxy group was determined to find less than 5 % of the charged amount and then the reaction mixture was cooled. The obtained compound was subjected to $^{13}C$ NMR analysis (AM 360 of Bruker Company; 90 MHz; dimethylsulfoxide standard), of which result is shown in Fig.1.

Example 2

The phenol ether of the present invention was prepared from the following ingredients, as generally described in Example 1.

| Ingredients | Parts by weight |
|---|---|
| Tamanol | 33 |
| Monoepoxy resin[1)] | 44 |
| n-Butanol | 10 |
| Methoxybutanol | 10 |
| Dimethylbenzylamine | 0.4 |

1) Available from Tohto Kasei Corporation as

The obtained compound was subjected to $^{13}C$ NMR, of which result is shown in Fig.1.

Example 3

(Synthesis of an epoxy resin having an amino group)

A reaction vessel was charged with 970 parts by weight of an epoxy resin having an epoxy equivalent 485 (available from Tohto Kasei Corporation as Epotohto YD-011) and 265 parts by weight of polycaprolactonediol (available from Union Carbide Corporation as PCP 0200). The mixture was heated to 100 °C in a nitrogen blanket and 0.46 parts by weight of dimethylbenzylamine was added. The reaction mixture was then heated to 130 °C and allowed to stand at this temperture for about one and half hours. The batch was cooled to 110 °C and 110 parts by weight of methyl isobutyl ketone was added. After adding 39.8 parts by weight of a 73 % methyl isobutyl ketone solution of methyl isobutyl diketimine of nonvolatile diethylenetriamine and 100 parts by weight of methyl isobutyle ketone, the batch was cooled to 70 °C, at which 53.1 parts by weight of diethylamine was added to adjust to 120 °C. The mixture was further kept for three hours to obtain the epoxy resin having an amino group.

(Synthesis of a blocked isocyanate resin)

A polyurethane cross-linking agent was prepared by mixing 291 parts by weight of a 80.20 (weight ratio) mixture of 2,4-/2,6-toluene diisocyanate with 218 parts by weight of 2-ethylhexanol in a dried nitrogen blanket while keeping the reaction vessel to 38 °C. The obtained product was allowed to stand for half hours at 38 °C and, after heating to 60 °C, 75 parts by weight of trimethylolpropane and 0.8 parts by weight of dibutyltin dilaurate were added to exotherm. The batch was maintained for one and half hours at 121 °C until isocyanato group is substantially consumed by Infrared analysis. The batch was diluted with 249 parts by weight of ethylene glycol monoethyl ether to adjust a solid content of 70 %.

(Preparation of a cationic aqueous paint)

A cationic aqueous paint was prepared from the following ingredients:

| Ingredients | Parts by weight |
|---|---|
| The epoxy resin having an amino group obtained above | 61 |
| The phenol ether of Example 1 | 10.5 |
| The blocked isocyanate resin obtained above | 29 |
| Deionized water | 76.3 |
| Lead acetate | 1.5 |
| Glacial acetic acid | 1.1 |
| Deionized water | 285.8 |
| Pigment paste obtained by a method of Japanese Patent Publication (examine) No. 4978/1979 | 37.0 |

A mixture of the epoxy resin having an amino group, the phenol ether and the blocked isocyanate resin was neutralized with glacial acetic acid and then diluted with the first deionized water. The second deionized water was added to the mixture and then the pigment paste was added to form a cationic aqueous paint.

Paint stability of the cationic aqueous paint was evaluated. The result is shown in Table 1.

Comparative Example 1

An aqueous paint was prepared as generally described in Example 3 with the exception that an initial reaction product of a resol type phenol which has a phenolic hydroxide group (Tamanol 722) was employed instead of the phenol ether of Example 1. Paints stability of the cationic aqueous paint was evaluated. The result is shown in Table 1.

Example 4

A cationic aqueous paint was prepared from the following ingredients as generally described in Example 3:

| Ingredients | Parts by weight |
|---|---|
| The epoxy resin having an amino group obtained above | 59.0 |
| The phenol ether of Example 1 | 43.7 |
| Deionized water | 75.5 |
| Glacial acetic acid | 1.4 |
| Deionized water | 284.4 |
| Pigment paste employed in Example 3 | 37.0 |

Paint stability of the cationic aqueous paint was evaluated. The result is shown in Table 1.

Comparative Example 2

An aqueous paint was prepared as generally described in Example 4 with the exception that an initial reaction product of a resol type phenol which has a phenolic hydroxide group (Tamanol 722) was employed instead of the phenol ether of Example 1. Paint stability of the cationic aqueous paint was evaluated. The result is shown in Table 1.

Example 5

A cationic aqueous paint was prepared from the following ingredients as generally described in Example 3:

| Ingredients | Parts by weight |
|---|---|
| The polybutadiene resin having an amino group[1] | 60.0 |
| The phenol ether of Example 2 | 35.0 |
| n-Butylized melamine[2] | 5 |
| Deionized water | 65.2 |
| Glacial acetic acid | 1.5 |
| Cobalt acetate | 0.3 |
| Deionized water | 266.5 |
| Pigment paste[3] | 36.5 |

[1] Available from Nippon Petrochemicals Company as EC 1800 NA.

[2] Available from Mitsui Toatsu Kagaku Company as Uvan 20SE-60.

[3] Pigment paste prepared by a method described in Japanese Patent Publication (unexamine) No.69635/1985

Paint stability of the cationic aqueous paint was evaluated. The result is shown in Table 1.

Comparative Example 3

An aqueous paint was prepared as generally described in Example 5 with the exception that an initial reaction product of a resol type phenol which has a phenolic hydroxide group (Tamanol 722) was employed instead of the phenol ether of Example 2. Paint stability of the cationic aqueous paint was evaluated. The result is shown in Table 1.

Table 1

| Example | Paint stability* |
|---|---|
| 3 | Good |
| 4 | Good |
| 5 | Good |
| Comparative Example 1 | Aggregation |
| 2 | Aggregation |
| 3 | Aggregation |

The paint was stirred for 24 hours at room temperature and filtered with 300 mesh net to evaluate aggregation.

Example 6

(Synthesis of a maleic polybutadiene resin)

| Ingredients | Parts by weight |
|---|---|
| Polybutadiene[1] | 1,000 |
| N-Methyl-N'-(1,3-dimethyl-butyl)-p-phenylenediamine | 10 |
| Maleic anhydride | 250 |
| Deionized water | 20 |
| Diethylamine | 0.5 |
| Propylene glycol | 100 |
| Ethylcellosolve | 340 |

[1] Mn 1500, vinyl 65 %, trans 14 %, cis 16 %; commercially available from Nippon Petrochemicals Company as Nisseki Polybutadine B-1500.

A reaction vessel was charged with 1,000 parts by weight of polybutadiene, 10 parts by weight on N-methyl-N-(1,3-dimethylbutyl)-p-phenylenediamine and 250 parts by weight of maleic anhydride. The mixture was heated to 190 to 200 °C with stirring to complete reaction. The reaction was terminated after about 5 hours. It was evidenced by a dimethylaniline coloring reaction. The reaction mixture was cooled to 100 °C and a mixture of 20 parts by weight of deionized water and 0.5 parts by weight of diethylamine was added dropwise over about 30 minutes. After completion of addition, the mixture was stirred for one hour to find an acid value of 140. Then, 100 parts by weight of propylene glycol was added to react for 3 hours at 110 °C until the acid value was 125. 340 parts by weight of ethylcellosolve was added and stirred for about one hour at 80 °C to terminate synthesis (solid content 80 %).

(Preparation of pigment paste)

125 parts by weight of the maleic polybutadiene resin was mixed with 13 parts by weight of triethylamine, to which 250 parts by weight of deionized water was added slowly to form a varnish having a nonvolatile content of about 26 %. The varnish was mixed with 150 parts by weight of titanium oxide, 50 parts by weight of lead silicate, 25 parts by weight of strontium chromate and 25 parts by weight of carbon black for about one hour by using a disper. The obtained mixture was ground using glass beads to less than 20 micron and filtered to remove the glass beads and then 1112 parts by weight of deionized water was added to terminate production(20 % solid content).

An anionic aqueous paint was prepared from the following ingredients as generally described in Example 3:

| Ingredients | Parts by weight |
|---|---|
| The maleic polybutadiene resins obtained above | 63.0 |
| The phenol ether of Example 1 | 67.0 |
| Triethylamine | 7.0 |
| Nonionic surfactant[1] | 1.0 |
| Cobalt naphthenate | 1.5 |
| Deionized water | 360.5 |
| Pigment paste obtained above | 100.0 |

[1] Available from Nippon Nyukazai Company as Newcol 710F

The anionic paint was electrocoated to a degreased SPCC dul steel panel (available from Nippon Test Panel Company) to form coatings having a thickness of 25 micron. The coated panel was cured for 20 minutes at 160 °C and subjected to a salt spray test and a pencil hardness test. The result is shown in Table 2.

Comparative Example 4

An anionic aqueous paint was prepared from the following ingredients as generally described in Example 6:

| Ingredients | Parts by weight |
|---|---|
| The maleic polybutadiene resins in Example 6 | 125.0 |
| Triethylamine | 14.0 |
| Nonionic surfactant[1] | 1.0 |

| | |
|---|---|
| Cobalt naphthenate | 1.5 |
| Deionized water | 358.5 |
| Pigment paste of Example 6 | 100.0 |

The same test was carried out as generally described in Example 6. The result is shown in Table 2.

Example 7

An anionic aqueous paint was prepared from the following ingredients as generally described in Example 6:

| Ingredients | Parts by weight |
|---|---|
| The maleic polybutadiene resins in Example 6 | 75.0 |
| Butylated melamine[1] | 40.0 |
| The phenol ether of Example 1 | 27.0 |
| Triethylamine | 8.0 |
| Nonionic surfactant of Example 6 | 1.0 |
| Cobalt naphthenate | 1.5 |
| Deionized water | 347.5 |
| Pigment paste obtained above | 100.0 |

[1] Available from Mitsui Toatsu Kagaku Company as Uvan 22R

The same test was carried out as generally described in Example 6. The result is shown in Table 2.

Comparative Example 5

An anionic aqueous paint was prepared from the following ingredients as generally described in Example 7:

| Ingredients | Parts by weight |
|---|---|
| The maleic polybutadiene resins in Example 6 | 94.0 |

| | |
|---|---|
| Butylated melamine in Example 7 | 50.0 |
| Triethylamine | 14.0 |
| Nonionic surfactant in Example 6 | 1.0 |
| Cobalt naphthenate | 1.5 |
| Deionized water | 358.5 |
| Pigment paste of Example 6 | 100.0 |

The same test was carried out as generally described in Example 6. The result is shown in Table 2.

Table 2

| Example | Salt spray test | Pencil hardness test |
|---|---|---|
| 6 | 3mm | H |
| 7 | 4mm | H |
| Comparative Example 4 | 8mm | HB |
| 5 | 10mm | F |

[1] The salt spray test was carried out according to JIS Z 2371 and a tape was peeled off after 72 hours to evaluate creepage width.

[2] The pencil hardness test was carried out according to JIS K 5400, Paragraph 6 to 14.

## Claims

1. A phenol ether characterized by a beta-hydroxyl group and has the following formula:

$$R^1 - O - \underset{R^5}{\overset{R^4}{C}} - \underset{OH}{\overset{R^3}{C}} - R^2 \qquad (1)$$

wherein $R^1$ is a residue of an initial reaction product of a resol type phenol resin from which a phenolic hydroxyl group is removed, $R^2$, $R^3$, $R^4$ and $R^5$ are respectively a saturated or unsaturated alkyl group having 1 to 30 carbon atoms, a saturated or unsaturated cycloalkyl group having 1 to 30 carbon atoms, or a substituted or unsubstituted aryl group having 6 to 20 carbon aroms ($R^3$ and $R^4$ may constitute a ring together).

2. A process for preparing the phenol ether of Claim 1 comprising reacting an initial reaction product of a resol type phenol resin with a monoepoxy compound in the presence of a basic catalyst.

3. The process according to Claim 2 wherein the initial reaction product of a resol type phenol resin has a number average molecular weight of less than 300.

4. The process according to Claim 2 or Claim 3 wherein the monoepoxy compound is monoglycidyl compound.

5. The process according to Claim 2 wherein the basic catalyst is amine compound.

6. A aqueous coating composition comprising the phenol ether of Claim 1 and a film forming resin.

7. A cationic electrocoating composition comprising the phenol ether of Claim 1 and a cationic resin.

8. An anionic electrocoating composition comprising the phenol ether of Claim 1 and an anionic resin.

Fig. 1

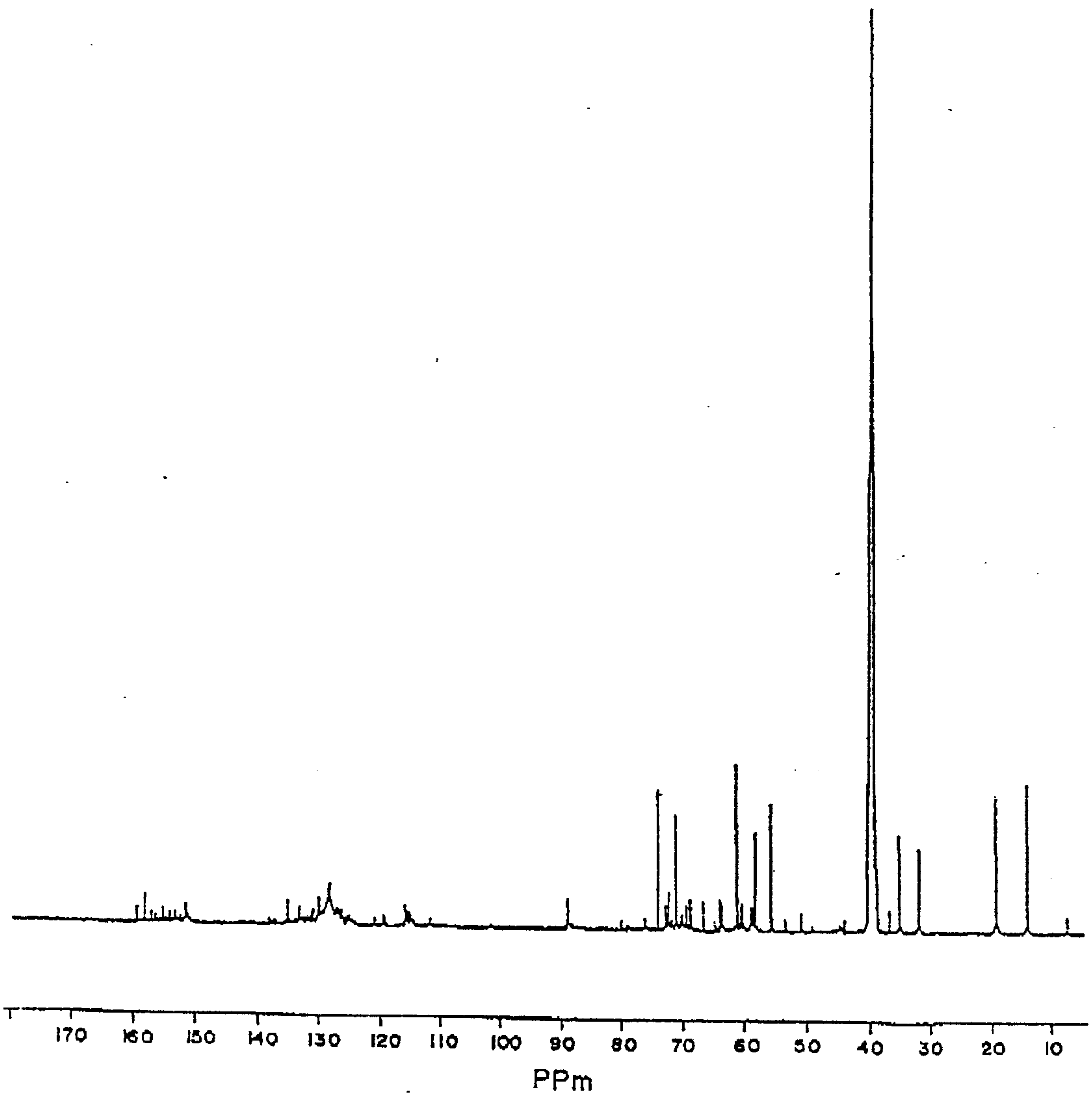

Fig. 2